# EUROPÄISCHE PATENTANMELDUNG

(11) **EP 0 979 655 A1**
(43) Veröffentlichungstag der Anmeldung: **16.02.2000**
(21) Anmeldenummer: 98114429.8
(22) Anmeldetag: 01.08.1998
(51) Int. Cl.: A61K 47/48, A61K 9/127

(54) **Mittel zur Behandlung von durch RNA-Viren, wie beispielsweise HIV verursachten Infenktionen**

(71) Anmelder: Roche Diagnostics GmbH, 68305 Mannheim (DE)
(72) Erfinder: Die Erfindernennung liegt noch nicht vor

(57) **Zusammenfassung**

Mittel zur Behandlung von Infektionen durch ribonukleinsäurehaltige Viren, dadurch gekennzeichnet, daß es aus zellartigen Vesikeln mit einer Lipidmembranhülle besteht, die an der Oberfläche spezifische Rezeptormoleküle für das die Infektion verursachende Virus aufweisen und ein oder mehrere Enzyme mit ribonukleolytischer Aktivität enthalten. Ferner betrifft die Erfindung ein Verfahren zur Herstellung des Mittels sowie ein Verfahren zur selektiven Inhibierung oder Zerstörung ribonukleinsäurehaltiger Viren in einer biologischen Probe.

## Beschreibung

Die Erfindung betrifft ein Mittel zur Behandlung von Infektionen durch ribonukleinsäurehaltige Viren sowie Verfahren zu dessen Herstellung und Verwendung. Im wesentlichen handelt es sich bei dem Mittel um zellartige, mit einer Lipidmembranhülle ausgestattete Vesikel, die an ihrer Oberfläche spezifische Rezeptormoleküle für das zu bekämpfende Virus aufweisen und ein Enzym mit ribonukleolytischer Aktivität, wie beispielsweise RNase enthalten.

Die klassische und in vielen Fällen immer noch wirksamste Methode zur Bekämpfung viraler Infektionen, die aktive Immunisierung mit entweder abgeschwächt infektiösen oder inaktivierten Viruspartikeln, versagt bei bestimmten Virus-Arten wie z.B. dem Influenza- oder dem humanen Immunschwäche-Virus (HIV), bedingt durch deren hohe genetische Variabilität, die sich unter anderem in ständig wechselnden Strukturen ihrer Oberflächen-Antigene äußert.

Auch gegenüber der Wirksamkeit von gegen das jeweilige Virus gerichtete Antikörpern oder von alternativ eingesetzten chemotherapeutischen Substanzen, die z.B. die Aktivität bestimmter viraler Replikationsenzyme, wie insbesondere Reverser Transkriptase, Integrase oder Protease hemmen, ist aufgrund dieser Variabilität mit der Entwicklung von Resistenzen zu rechnen (vgl. z.B. "HIV und AIDS", K.E. Nye/J.M. Parkin, Spektrum Akademischer Verlag, Heidelberg 1995, S. 91). Darüber hinaus ist von Nachteil, daß ― zumindest bislang ― mit derartigen Substanzen, insbesondere bei HIV-Infektionen bzw. AIDS, lediglich krankheitsverzögernde Wirkungen erreicht werden.

Es besteht daher nach wie vor ein Bedarf an antiviralen Mitteln, die sich möglichst spezifisch gegen entweder nicht oder wenig wandelbare Virus-Strukturelemente (sogenannte konservierte Strukturelemente) richten oder gegen andere, für die Vermehrungsfähigkeit erforderliche Virus-Bestandteile, die auch bei Veränderungen ihrer Zusammensetzung ihre Suszeptibilität gegenüber dem therapeutischen Mittel uneingeschränkt beibehalten.

Ein Bestandteil des letzteren Typs ist insbesondere das genetische Material des Virus selber, d.h. je nach Virus-Typ entweder Desoxyribonukleinsäure oder ― wie z.B. im Falle des Influenza-, des Rhino- oder des HI-Virus ― Ribonukleinsäure (RNA).

Dieses Material läßt sich unter physiologischen Bedingungen auf besonders einfache Weise und unabhängig von seiner Nukleinsäurezusammensetzung bzw. Basen-Sequenz enzymatisch, insbesondere durch Einwirkung von Nukleasen, bevorzugt durch Endonukleasen, wie entweder Desoxyribonukleasen ("DNasen") oder Ribonukleasen ("RNasen"), abbauen und damit inaktivieren.

Insbesondere handelt es sich bei vielen der bekannten RNasen um hochaktive und gleichzeitig ungewöhnlich stabile Enzyme mit in der Regel sehr niedrigem Molekulargewicht (ca. 10-30 kDa) dar, was sie für den genannten Zweck, sofern es sich beim genetischen Material des Virus um RNA handelt, besonders geeignet macht.

Dabei stellt sich jedoch das Problem, auf welche Weise die virale RNA, die entweder im nativen Virus durch eine Protein-Capsid- sowie zum Teil zusätzlich durch eine Lipid-Membranhülle geschützt vorliegt, oder die sich innerhalb vom Virus befallener Körperzellen befindet, einem Angriffdurch solche RNasen ausgesetzt werden kann, ohne daß andere bzw. nichtinfizierte Körperzellen durch eine unspezifische Einwirkung des Enzyms geschädigt werden.

Die der Erfindung zugrunde liegende Aufgabe besteht somit darin, Nukleasen wie insbesondere RNasen als antivirales Mittel in einer Form darzubieten, die ihren direkten Kontakt mit der Ribonukleinsäure von Virus-Partikeln oder der Ribonukleinsäure von mit dem entsprechenden Virus infizierten Körperzellen ermöglicht, ohne daß sie unspezifische cytotoxische Effekte entfalten kann und/oder daß die Nuklease- bzw. RNase-Form ausreichend stabil bleibt, d.h. nicht zu schnell inaktiviert bzw. aus dem Körper eliminiert wird.

Diese Aufgabe wird erfindungsgemäß dadurch gelöst, daß man eine RNase in Zellen oder zellartige Vesikel mit (Proteo-)Lipidmembranhüllen einschließt, die auf ihrer Oberfläche bzw. in die Membranhülle integriert mit für das zu bekämpfende Virus spezifischen Rezeptormolekülen ausgestattet sind bzw. anschließend ausgestattet werden. Zur Behandlung von durch HIV bzw. insbesondere durch HIV-1 verursachten Infektionen werden als Rezeptormoleküle bevorzugt CD4 sowie ein oder mehrere Vertreter aus der Gruppe der Chemokin-Rezeptorproteine bzw. CC- oder CXC-Rezeptormoleküle, wie insbesondere CCR2b-, CCR3-, CXCR4 und/oder CCR5 (vgl. z.B. Science vol. 280, 1833-1834 (1998); D. Hammache et al., J. Biol. Chem. vol. 273, 7967-7971 (1998)) und/oder aktive Bruchstücke bzw. Derivate davon verwendet. Im Falle des Influenza-Virus wird bevorzugt ein Glykoprotein mit endständiger N-Acetylneuraminsäure verwendet. Für Rhinoviren dient als Rezeptormolekül das sogenannte interzelluläre Adhäsionsmolekül 1 ("ICAM-1").

Weiterhin können die mit denjeweiligen Rezeptormolekülen ausgestatteten Zellen oder zellartigen Vesikel anstelle von, oder, was bevorzugt ist, zusätzlich zu RNase eine oder mehrere Substanzen mit RNase-Inhibitor-neutralisierender Wirkung und/oder RNase-Aktivatoren wie z.B. Oligoadenylate mit 2'→5'-Internucleotid-Verknüpfung (Aktivatoren für RNase L, vgl. z.B. R.H. Silverman, in "Ribonucleases" (G.D'Alessio and J.F. Riordan, Hrsg.), Academic Press 1997, 516-551) und/oder zweiwertige Metallionen wie insbesondere Mg²⁺ enthalten, um entweder die jeweils zugesetzte RNase vor einer etwaigen Inaktivierung durch intrazelluläre RNase-Inhibitoren zu schützen und/oder von vornherein in Körperzellen enthaltene, durch RNase-Inhibitoren inaktivierte oder ansonsten in inaktiver Form vorliegende RNase-Moleküle zu reaktivieren.

Mit dem erfindungsgemäßen Mittel gelingt sowohl die selektive Zerstörung bzw. Inaktivierung von freien Viruspartikeln als auch von vom entsprechenden Virus infizierten Körperzellen.

Im ersten Fall fungiert das Vesikel wie die natürliche Virus-Zielzelle, d.h. durch Rezeptor-vermittelte Bindung des Virus an die Vesikel-Oberfläche mit anschließender Membranfusion und intravesikulärer Freisetzung seines genetischen Materials (RNA), jedoch ohne daß es zur Virus-Replikation, sondern vielmehr zur Zerstörung der viralen Ribonukleinsäure durch die in den Vesikeln enthaltene RNase kommt.

Im zweiten Fall kommt es zu einer sogenannten Superinfektion, indem sich das Rezeptor-dotierte, RNase-haltige Vesikel an die aufder Oberfläche der infizierten Zelle exprimierten viralen Hüll(glyco-)proteine anheftet, anschließend ebenfalls mit der Zelle fusioniert (syncytien- bzw. Riesenzell-Bildung) und seinen Inhalt, d.h. RNase sowie gegebenenfalls zusätzlich RNase-Inhibitor-neutralisierende Substanz(en) und/oder RNase-Aktivatoren im Zellinnern freisetzt. Dadurch wird nicht nur virale, sondern auch andere zelluläre RNA (mRNA, tRNA und rRNA) zerstört, was den zusätzlichen Vorteil hat, daß die infizierte Zelle gänzlich zugrunde geht und somit in ihr auch keine weitere Virus-Replikation mehr stattfinden kann.

Gleichzeitig wird durch den Einschluß des Enzyms verhindert, daß die RNase in unerwünschter Weise mit nichtinfizierten oder anderen Zelltypen in Wechselwirkung tritt oder vorzeitig aus dem Organismus eliminiert wird.

Als RNasen sind sowohl native RNasen geeignet, als auch Di- oder Polymere davon, oder/und di- oder polymere RNase-Muteine, oder/und enzymatisch aktive Spaltstücke, Derivate bzw. Gemische. Besonders bevorzugt werden erfindungsgemäß solche RNasen eingesetzt, die nicht durch natürliche RNase-Inhibitoren prokaryontischer bzw. Säuger-Zellen hemmbar sind, wie insbesondere RNase I (z.B. aus E.coli), Seminal-RNase bzw. durch SH-Verbindungen nicht spaltbare Muteine von dimerer Seminal- bzw. Pankreas-RNase (vgl. z.B. EPA 0 668 349; J.-S. Kim et al., J. Biol. Chem. vol. 270, 31097-31102 (1995)) und/oder Onconase.

Um etwaige Immunreaktionen gegen die genannten RNasen bzw. RNase-Derivate oder Spaltstücke zu verhindern oder zu reduzieren, insbesondere wenn sie nichthumanen Ursprungs sind, können sie des weiteren durch kovalente Verknüpfung mit bestimmten Substanzen wie z.B. Polyethylenglycol modifiziert sein (vgl. z.B. T.-D. Brumeanu et al., J. Chromatogr. vol. 696, 219-225 (1995); R. Bovara et al., Biotechnol. Bioeng. vol. 54, 50-57 (1997)).

Als RNase-Inhibitor-neutralisierende Substanzen können beispielsweise gegen RNase-Inhibitor(en) gerichtete Antikörper bzw. (Fab-)Fragmente solcher Antikörper und/oder mit Thiol-Gruppen reagierende Substanzen verwendet werden.

Als Aktivatoren für zelluläre RNase L werden Di- bis Pentamere von 2'→5'-Oligoadenylaten bevorzugt, insbesondere das Trimer ppp5^{'}(A2'p5')₂A (vgl. R.H. Silverman, in: "Ribonucleases" (G.D'Alessio and J.F. Riordan, Hrsg.), Academic Press, 1997, 518).

Als Lipidvesikel eignen sich Liposomen, insbesondere unilamellare Liposomen, Plasmamembran-Vesikel (PMV), oder Erythrozyten. Alternativ können auch andere Körperzellen als RNasehaltige Vesikel dienen. In diesem Fall sind solche Zellen besonderes bevorzugt, die von vornherein CD4- und CCR5- und/oder CXCR4- sowie gegebenenfalls CCR2b- und/oder CCR3-Rezeptoren aufweisen, wie beispielsweise periphere mononukleare Blutzellen bzw. T-Lymphozyten oder Macrophagen.

Die Herstellung von mit bestimmten Rezeptormolekülen oberflächendotierten Vesikeln ist dem Fachmann seit längerem bekannt (Cudd et al., J. AIDS vol. 3, 109-114 (1990); Zeira et al. Proc. Natl. Acad. Sci. USA vol. 88, 4409-4413 (1991); WO 94/04677; sowie Endres et al., Science vol. 278, 1442-1464 (1997)).

Ferner ist bekannt, daß entsprechende Vesikel geeignet sind, antivirale Agenzien spezifisch in virusinfizierte Körperzellen einzuschleusen; in WO 94/04677 wird insbesondere erwähnt, daß ein solches Agens unter anderem ein Enzym sein kann. Ein konkreter Hinweis, um welche Substanz(en) bzw. Enzym(e) es sich im einzelnen handeln könnte, ist dem Stand der Technik dagegen nicht zu entnehmen. Insbesondere findet sich in keiner der bekannten Literaturstellen ein Hinweis auf eine mögliche Verwendung von Nukleasen, insbesondere Ribonukleasen und darüber hinaus nicht auf RNase-Inhibitor-neutralisierende und/oder RNase-aktivierende Substanzen.

In der Patentschrift EP 0 789 715 sind unter anderem antivirale Liposomen beschrieben, die auf ihrer Oberfläche ein virusspezifisches Bindemolekül ("target-binding moiety") und eine oder mehrere Hydrolasen zur Verdauung von viralen Bestandteilen enthalten, wobei Bindemolekül und Hydrolase jeweils kovalent mit der liposomalen Lipidschicht verknüpft sind. Für den Fall von anti-HIV-Liposomen wird als Bindemolekül bevorzugt das lösliche CD4-Fragment, als Hydrolase kann beispielsweise Ribonuclease verwendet werden. Als weitere, an die Oberfläche verknüpfte Hydrolasen werden erfindungsgemäß Glycosidase, Phospholipase(n), Lipase und/oder Cholesterin-Esterase bevorzugt eingesetzt. Eine oder mehrere der genannten Hydrolasen können sich zusätzlich auch im Inneren der Liposomen befinden.

Insbesondere wird in EP 0 789 715 darauf hingewiesen, daß viele Viren wie unter anderem HIV von einer Lipidhülle, bestehend aus Phospholipiden, Triglyceriden und Cholesterin-Estern, umgeben sind, und daß deswegen zusätzlich drei Enzymarten, d.h. Phospholipasen, Lipasen und Cholesterin-Esterasen, auf der Oberfläche der Liposomen zugegen sein sollten, um die virale Lipidschicht abzubauen und das Protein-Core, sowie nach dessen spontaner Auflösung im (Blut-)Plasma, die RNA freizusetzen. Schließlich wird im weiteren ausdrücklich betont, daß die erfinderische Idee im Kern darauf abzielt, das Virus außerhalb der Zelle aufzulösen bzw. dort seine RNA zu zerstören. Diese Feststellung ist auch insofern offensichtlich, als ein seiner Lipidhülle beraubtes HI-Virus kaum in der Lage sein dürfte, mit seiner Wirtszelle oder auch dem antiviralen Liposom zu fusionieren. Letzteres wird auch deswegen nicht erfolgen können, da in EP 0 789 715 Patent als liposomales HIV-Erkennungsmolekül lediglich CD4 bzw. das lösliche Fragment von CD4, nichtjedoch weitere, für die Virus-Fusion erforderlichen Co-Rezeptoren wie z.B. CCR5 bzw. CXCR4, erwähnt sind.

Somit hat das Verfahren nach EP 0 789 715 den erheblichen Nachteil, daß bestenfalls freie Viren, nichtjedoch vom Virus infizierte Körperzellen angegriffen bzw. zerstört werden und somit bei Absetzen der Behandlung eine erneute Virusvermehrung zu erwarten ist.

Im Gegensatz dazu gelingt mit dem erfindungsgemäßen Mittel gleichermaßen die Zerstörung freier Virus-Partikel wie auch vom Virus befallener Körperzellen, so daß auch nach Beendigung seiner Anwendung keine weitere Virusvermehrung mehr stattfinden kann.

Ein weiterer Gegenstand der Erfindung ist ein Verfahren zur Herstellung eines Mittels bestehend aus zellartigen Vesikeln mit einer Lipidmembranhülle, die an der Oberfläche spezifische Rezeptormoleküle für das jeweilige ribonukleinsäurehaltige Ziel-Virus aufweisen und ein oder mehrere Enzyme mit ribonukleolytischer Aktivität enthalten. Im wesentlichen wird dabei zunächst ein aus verschiedenen Lipidkomponenten, wie z.B. Phosphatidylcholinen, Phosphatidylglycerin und gegebenenfalls Sialoganggliosid, bestehendes Lipidgemisch hergestellt, welches anschießend mit (einem) Enzym(en) mit ribonukleolytischer Aktivität, z.B. RNase I, sowie mit spezifischen Rezeptormolekülen, z.B. CD4, CCR5 und CXCR4, inkubiert wird. Die resultierende und entsprechend der Größe der Vesikel bestimmte Suspension stellt nach Resuspenieren und Waschen in physiologischer Kochsalzlösung die gebrauchsmäßige Liposomenpräparation dar.

Für den Fall, daß das erfindungsgemäße Mittel aufBasis von ursprünglich CD4- sowie CCR5- und/oder CCR2b- und/oder CCR3- und/oder CXCR4-Rezeptoren tragenden Zellen ausgeht, werden die Ribonuklease(n) oder/und davon abgeleitete enzymatisch aktiven Derivate sowie gegebenenfalls zusätzlich eine oder mehrere RNase-Inhibitor-neutralisierende Substanzen und/oder RNase-Aktivatoren in diese Zellen einschleust, indem man die Zellen entweder
a) einer hypotonischen Schockbehandlung mittels einer niedermolekularen wäßrigen Pufferlösung, wobei RNase(n) oder/und davon abgeleitete enzymatisch aktive Derivate sowie gegebenenfalls eine oder mehrere RNase-Inhibitor-neutralisierende Substanzen und/oder RNase-Aktivatoren im niederosmolaren Puffer zugegen sind,
b) einer Fusion mit einer oder mehreren RNasen oder/und davon abgeleiteten enzymatisch aktiven Derivaten sowie gegebenenfalls einer oder mehreren RNase-Inhibitor-neutralisierenden Substanzen und/oder RNase-Aktivatoren beladenen Erythrozyten-Ghosts bzw. Liposomen, oder
c) einer Elektroinsertion in einem wäßrigen, gepufferten Medium, enthaltend eine oder mehrere RNasen oder/und davon abgeleitete enzymatisch aktive Derivate sowie gegebenenfalls eine oder mehrere RNase-Inhibitor-neutralisierenden Substanzen und/oder RNase-Aktivatoren unterwirft.

Ein weiterer Gegenstand ist ein Verfahren zur spezifischen Erkennung und vollständigen oder teilweisen selektiven Inhibierung oder Zerstörung von ribonukleinsäurehaltigen Viren in einer biologischen Probe, dadurch gekennzeichnet, daß zellartige Vesikel bestehend aus einer Lipidmembranhülle, die an der Oberfläche für das Virus spezifische Rezeptormoleküle aufweisen und ein oder mehrere Enzyme mit ribonukleolytischer Aktivität und gegebenenfalls eine oder mehrere RNase-Inhibitor-neutralisierende Substanzen und/oder RNase-Aktivatoren enthalten, mit der Probe vermischt und inkubiert werden.

Die folgenden Beispiele erläutern die Erfindung weiter.

### Beispiel 1:

### Herstellung RNase-haltiger, mit DC4-, CCR5- und CXCR4-Rezeptoren membrandotierter Liposomen

In einem Volumen von 10 ml CHCl₃ werden 35 mg eines Lipidgemisches mit der folgenden Zusammensetzung aufgelöst:

| Lipid | Teile |
|---|---|
| Cholesterin | 2,6 |
| Di-myristoyl-phosphatidylcholin | 1 |
| Di-palmitoyl-phosphatidylcholin | 1 |
| Di-stearyl-phosphatidylcholin | 1 |
| Di-myristoyl-phosphatidylglycerin | 0,3 |
| Monosialogangliosid-G_{M3} | 0,1 |

Nach Entfernen des CHCl₃ im Rotationsverdampfer werden zu dem Lipidfilm 2 ml einer neutralen wäßrigen Lösung, enthaltend RNase I (10 mg) sowie die CD4-, CCR5- und CXCR4-Rezeptoren (je 2 mg), hinzugefügt. Die Mischung wird mit einem Vortex-Rührer homogenisiert und anschließend 1 h in einem Wasserbad bei 37°C geschüttelt.

Die Protein-Lipid-Suspension wird bei 20°C in eine Ultrafiltrations-Zelle (Amicon) gegeben, und große unilamellare Vesikel (2-3 µm ⌀) werden durch sukzessives Auspressen durch Polycarbonat-Membranfilter mit ahnehmender Porengröße (10/5/2 µm) gebildet.

Die resultierende Suspension wird resuspendiert und stellt nach zweimaligem Waschen mit Na-Phosphat-Puffer, 5 mmol/l, NaCl, 145 mmol/l, pH 7,4 (PBS pH 7,4) (je 20 min Zentrifugation bei 16.000 Upm) die gebrauchsfertige Liposomenpräparation dar.

Die Zusammensetzung und Herstellung der oben genannten Liposomen stellt, insbesondere was ihre Lipid-Bestandteile betrifft, nur eine von vielen Möglichkeiten dar, wie dem Fachmann an sich geläufig ist; weitere Liposomenpräparationen sind z.B. in A. Cudd et al., J. of Aquired Immune Deficiency Syndrome vol. 3, 109-114 (1990) und in N. Dusserre et al., AIDS vol. 9, 833-841 (1995) beschrieben. Ferner ist in diesem Zusammenhang auf den Übersichtsartikel von G. Gregoriadis, Trends in Biotechnology vol. 13, 527-537 (1995) zu verweisen.

Die Konzentrationen der eingesetzten RNase(n) bzw. RNase-Derivate sowie der CD4- und CC- bzw. CXC-Rezeptoren in weiteren Grenzen variiert werden, von etwa 0,001 mg/ml bis praktisch hin zu ihrer Löslichkeitsgrenze im wäßrigen Inkubationsmedium.

### Beispiel 2:

### Herstellung RNase-haltiger, mit CD4-, CCR5- und CXCR4-Rezeptoren membrandotierter Erythrozytenghosts

Aus menschlichem Blut isolierte, gepackte Erythrozyten (1 ml) werden bei 1-4°C rasch und intensiv mit ca. 40 ml Na-Phosphat-Puffer, 5 mmol/l, pH 8,0, enthaltend MgSO₄, 1 mmol/l, RNase I (5 mg/ml) sowie CD4-, CCR5- und CXCR4-Rezeptoren (je 0,2 mg/ml) vermischt und nach ca. 30 min bei 22.000 g 10 min lang abzentrifugiert.

Alternativ können gepackte Erythrozyten (1 ml) bei 1-4°C rasch und intensiv mit ca. 40 ml Na-Phosphat-Puffer, 5 mmol/l, pH 8,0, enthaltend RNase I (5 mg/ml) sowie CD4-, CCR5- und CXCR4-Rezeptoren (je 0,2 mg/ml) vermischt und anschließend 10 min bei 22.000 g abzentrifugiert werden. Das Pellet wird in 40 ml PBS pH 8,0, enthaltend RNase I (5 mg/ml), resuspendiert und für 40 min bei 37°C inkubiert. Die so erhaltenden, RNase-haltigen und HIV-Rezeptor-dotierten Ghosts werden zwei weitere Male mit Na-Phosphat, 5 mmol/l (pH 8.0), NaCl, 145 mmol/l (PBS pH 8,0) gewaschen.

Schließlich können auch isolierte Erythrozyten (1 ml) zunächst einer kurzzeitigen hypotonischen Schockbehandlung mit z.B. ca. 40 ml Na-Phosphat-Puffer, 5 mmol/l, pH 8,0, enthaltend MgSO₄, 1 mmol/l, und RNase I (5 mg/ml), unterworfen und erst anschließend an ihre Versiegelung bzw. Isolierung (s.o.) mit den CD4- und CC- bzw. CXC-Rezeptoren, z.B. durch Elektroinsertion analog zu z.B. M. Zeira et al., Proc. Natl. Acad. Sci. vol. 88, 4409-4413 (1991), dotiert werden.

Bezüglich der Konzentrationen von RNase sowie der CD4- und CC- bzw. CXC-Rezeptoren gelten die im Beispiel 1 gemachten Angaben.

### Beispiel 3:

### Herstellung RNase-haltiger peripherer mononuklearer Blut-Zellen (= peripheral blood mononuclear cells. "PBMC")

Die Herstellung erfolgt analog der der RNase-haltigen, mit CD4-, CCR5- und CXCR4-Rezeptoren membrandotierten Erythrozytenghosts nach Beispiel 2, nur daß die Lyse-Inkubation mit Na-Phosphat-Puffer, 5 mmol/l, pH 8,0, enthaltend MgSO₄, 1 mmol/l, und RNase I (5 mg/ml), bzw. alternativ mit 40 ml Na-Phosphat-Puffer, 5 mmol/l, pH 8,0, enthaltend RNase I (5 mg/ml), in beiden Fällen ohne CD4-, CCR5- und CXCR4-Rezeptoren durchgeführt wird, da die PBMC's bereits von vornherein die entsprechenden Rezeptoren membrangebunden aufweisen.

Bei der Verwendung von bereits ursprünglich mit virusspezifischen Rezeptoren ― wie für das HI-Virus insbesondere mit CD4, CCR5 und/oder CXCR4 sowie gegebenenfalls CCR2b bzw. CCR3 ― ausgestatteten Zellen, kann die Einschleusung von RNase (sowie gegebenenfalls weiterer Hilfskomponenten wie z.B. RNase-Inhibitor-neutralisierender und/oder RNase-aktivierender Substanzen) auch mit anderen Methoden wie z.B. durch Fusion mit durch spezifische Makromoleküle bzw. Proteine beladenen Erythrozytenghosts oder Liposomen, oder durch Elektroinsertion erfolgen, die dem Fachmann prinzipiell bekannt sind (s. z.B. "Methods in Enzymology", Academic Press 1993, Band 221, Seiten 306-376).

## Patentansprüche

1. Mittel zur Behandlung von Infektionen durch ribonukleinsäurehaltige Viren, dadurch gekennzeichnet, daß es aus zellartigen Vesikeln mit einer Lipidmembranhülle besteht, die an der Oberfläche spezifische Rezeptormoleküle für das die Infektion verursachende Virus aufweisen und ein oder mehrere Enzyme mit ribonukleolytischer Aktivität enthalten.

2. Mittel nach Anspruch 1, dadurch gekennzeichnet, daß es sich bei dem oder den Enzymen um Ribonukleasen oder/und davon abgeleitete enzymatisch aktive Derivate handelt.

3. Mittel nach Ansprüchen 1 und 2, dadurch gekennzeichnet, daß zusätzlich eine oder mehrere RNase-Inhibitor-neutralisierende Substanzen und/oder RNase-Aktivatoren enthalten sind.

4. Mittel nach Ansprüchen 1 bis 3, dadurch gekennzeichnet, daß zur Behandlung von Infektionen durch das humane Immunschwäche-Virus als Rezeptormoleküle ein CD4-Molekül sowie ein oder mehrere Vertreter aus der Gruppe der CC- oder CXC-Rezeptormoleküle und/oder Bruchstücke bzw. Derivate davon mit HIV-Rezeptorfunktion verwendet werden.

5. Verfahren zur Herstellung eines Mittels gemäß einem der Ansprüche 1 bis 4 dadurch gekennzeichnet, daß man für die zellartigen Vesikel mit Lipidmembranhülle von Liposomen, Plasmamembranvesikeln, Erythrozyten und/oder sonstigen Körperzellen ausgeht, diese mit mindestens einem Enzym mit ribonukleolytischer Aktivität inkubiert und die resultierende Suspension isoliert.

6. Verfahren nach Anspruch 5, dadurch gekennzeichnet, daß es sich bei den Körperzellen um isolierte, CD4- sowie CCR5- und/oder CCR2b- und/oder CCR3- und/oder CXCR4-Rezep-toren tragende Zellen handelt.

7. Verfahren nach Anspruch 6, dadurch gekennzeichnet, daß es sich bei den CD4- sowie CCR5- und/oder CCR2b- und/oder CCR3- und/oder CXCR4-Rezeptoren tragenden Zellen um periphere mononukleare Blutzellen oder diesbezügliche Subtypen wie beispielsweise T-Lymphozyten und/oder Makrophagen handelt.

8. Verfahren nach Anspruch 5, dadurch gekennzeichnet, daß man im Falle der Verwendung von Liposomen als zellartige Vesikel diese in Gegenwart einer wäßrigen Lösung, enthaltend eine oder mehrere Ribonukleasen oder/und davon abgeleitete enzymatisch aktive Derivate, einen CD4-Rezeptor sowie einen oder mehrere Vertreter aus der Gruppe der CC- und/oder CXC-Rezeptormoleküle oder/und diesbezügliche Bruchstücke bzw. Derivate mit HIV-Rezeptorfunktion sowie gegebenenfalls zusätzlich eine oder mehrere RNase-Inhibitor-neutralisierende Substanzen und/oder RNase-Aktivatoren, herstellt.

9. Verfahren nach Anspruch 5, dadurch gekennzeichnet, daß man im Falle der Verwendung von Erythrozyten als zellartige Vesikel diese einer hypotonischen Schockbehandlung mittels einer niederosmolaren wäßrigen Pufferlösung, enthaltend eine oder mehrere Ribonukleasen oder/und davon abgeleitete enzymatisch aktive Derivate, einen CD4-Rezeptor sowie einen oder mehrere Vertreter aus der Gruppe der CC- und/oder CXC-Rezeptormoleküle oder/und diesbezügliche Bruchstücke bzw. Derivate mit HIV-Rezeptorfunktion sowie gegebenenfalls eine oder mehrere RNase-Inhibitor-neutralisierende Substanzen und/oder RNase-Aktivatoren, unterwirft.

10. Verfahren zur spezifischen Erkennung und vollständigen oder teilweisen selektiven Inhibierung oder Zerstörung von ribonukleinsäurehaltigen Viren in einer biologischen Probe, dadurch gekennzeichnet, daß zellartige Vesikel bestehend aus einer Lipidmembranhülle, die an der Oberfläche für das Virus spezifische Rezeptormoleküle aufweisen und ein oder mehrere Enzyme mit ribonukleolytischer Aktivität und gegebenenfalls eine oder mehrere RNase-Inhibitor-neutralisierende Substanzen und/oder RNase-Aktivatoren enthalten, mit der Probe vermischt und inkubiert werden.
